(19) Europäisches Patentamt
European Patent Office
Office européen des brevets

(11) EP 2 599 498 A1

(12) **EUROPEAN PATENT APPLICATION**

(43) Date of publication:
**05.06.2013 Bulletin 2013/23**

(21) Application number: **13157478.2**

(22) Date of filing: **25.06.2008**

(51) Int Cl.:
***A61K 45/00*** *(2006.01)* ***A61K 31/426*** *(2006.01)*
***A61P 27/02*** *(2006.01)*

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **25.06.2007 JP 2007166989**

(62) Document number(s) of the earlier application(s) in accordance with Art. 76 EPC:
**08790612.9 / 2 172 221**

(71) Applicant: **R-Tech Ueno, Ltd.**
**Tokyo 100-0011 (JP)**

(72) Inventors:
- **Mashima, Yukihiko**
**Tokyo, 100-0011 (JP)**
- **Kurono, Naomi**
**Tokyo, 100-0011 (JP)**
- **Yoshikawa, Nami**
**Tokyo, 100-0011 (JP)**

(74) Representative: **Duckworth, Timothy John**
**J A Kemp**
**14 South Square**
**Gray's Inn**
**London WC1R 5JJ (GB)**

Remarks:
This application was filed on 01-03-2013 as a divisional application to the application mentioned under INID code 62.

(54) **Composition for ophthalmic disease associated with hypoxia or ischemia**

(57) According to the present invention, a composition containing a VAP-1 inhibitor as an active ingredient, which is effective for ophthalmic diseases associated with hypoxia or ischemia can be provided. According to the present invention, moreover, an angiogenesis inhibitor that suppresses pathologic angiogenesis associated with hypoxia or ischemia can be provided.

EP 2 599 498 A1

## Description

Technical Field

The present invention relates to a composition for an ophthalmic disease associated with hypoxia or ischemia and an angiogenesis inhibitor.

Background Art

The vascular adhesion protein-1 (hereinafter to be abbreviated as VAP-1) is amine oxidase (semicarbazide sensitive amine oxidase, SSAO) abundantly existing in human plasma, which shows a remarkably increased expression in vascular endothelium and vascular smooth muscle in the inflammatory lesion. Although the physiological role of VAP-1 has not been elucidated until recently, VAP-1 gene was cloned in 1998, and VAP-1 was reported to be a membrane protein which, as an adhesion molecule, controls rolling and migration of lymphocytes and NK cells under the expression control of inflammatory cytokine. Although amine to be the substrate is unknown, it is considered to be methylamine produced in any part in the living body. It is also known that hydrogen peroxide and aldehyde produced due to the intramolecular amine oxidase activity are important factors for adhesion activity.
In addition, it is described that a thiazole derivative having a particular structure, which is a VAP-1 inhibitor, is used for the prophylaxis or treatment of VAP-1 related diseases such as macular edema, vascular hyperpermeable disease and the like (patent documents 1 - 4). Although the VAP-1 inhibitor is known to be useful for the VAP-1 related diseases, its usefulness for ophthalmic diseases associated with hypoxia or ischemia is not known.

patent document 1: WO2004/067521
patent document 2: WO2004/087138
patent document 3: W02006/011631
patent document 4: WO2006/028269

Disclosure of the Invention

Problems to be Solved by the Invention

The present invention provides a new use of a VAP-1 inhibitor and, for example, aims to provide a composition effective for ophthalmic diseases associated with hypoxia or ischemia and a composition effective for angiogenesis suppression. Furthermore, it aims to provide a treatment method of ophthalmic diseases associated with hypoxia or ischemia, a method of suppressing angiogenesis and the like. Means of Solving the Problems
The present inventors have conducted intensive studies and found that a VAP-1 inhibitor is effective for the treatment of ophthalmic diseases associated with hypoxia or ischemia and suppression of angiogenesis, which resulted in the completion of the present invention.
Accordingly, the present invention provides the following.
[1] A composition for an ophthalmic disease associated with hypoxia or ischemia, comprising a VAP-1 inhibitor as an active ingredient.
[2] The composition of [1], wherein the aforementioned VAP-1 inhibitor is a compound represented by the following formula (I):
$$\mathbf{R^1\text{-}NH\text{-}X\text{-}Y\text{-}Z} \qquad \mathbf{(I)}$$

wherein
$R^1$ is acyl;
X is a divalent residue induced from optionally substituted thiazole;
Y is the formula: $Y^1$-$X^2$-$Y^3$
wherein $Y^1$ is a bond, lower alkylene, lower alkenylene, lower alkynylene, $-(CH_2)_n-O-$, $-(CH_2)_n-NH-$, $-(CH_2)_n-CO-$ or $-(CH_2)_n-SO_2-$ (wherein n is an integer of 0 to 6);
$Y^2$ is a bond, -O-, -NH-, -CO- or $-SO_2-$;
$Y^3$ is a bond, lower alkylene, lower alkenylene or lower alkynylene, provided that when $Y^1$ is $-(CH_2)_n-O-$, then $Y^2$ is not -O-, -NH- or $-SO_2-$, when $Y^1$ is $-(CH_2)_n-NH-$, then $Y^2$ is not -O- or -NH-, when $Y^1$ is $-(CH_2)_n-CO-$, then $Y^2$ is not -CO-, and when $Y^1$ is $-(CH_2)_n-SO_2-$, then $Y^2$ is not -O- or $-SO_2-$(wherein n is as defined above);
Z is the formula:

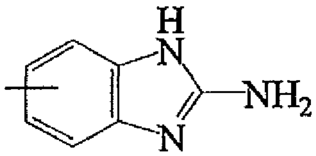 , 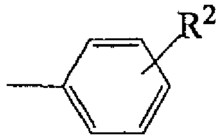 or 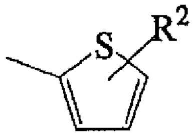

wherein $R^2$ is the formula: -A-B-D-E
wherein
A is a bond, lower alkylene, $-NR^{2a}-$ or $-SO_2-$ wherein $R^{2a}$ is hydrogen, lower alkyl or acyl;
B is a bond, lower alkylene, -CO- or -O-;
D is a bond, lower alkylene, $-NR^{2b}-$ or $-CH_2NH-$ wherein $R^{2b}$ is hydrogen, lower alkyl, alkoxycarbonyl or acyl; and
E is optionally substituted amino, $-N=CH_2$,
**[0010]**

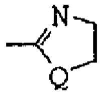 or 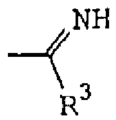

wherein
Q is -S- or -NH-;
$R^3$ is hydrogen, lower alkyl, lower alkylthio or $-NH-R^4$ wherein $R^4$ is hydrogen, $-NH_2$ or lower alkyl, or a derivative thereof, or a pharmaceutically acceptable salt thereof.
[3] The composition of [2], wherein, in the formula (I), Z is the formula (II):
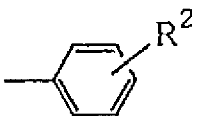

(I I)

wherein
$R^2$ is the formula:
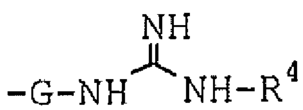

wherein G is a bond, $-NHCOCH_2-$ or lower alkylene; $R^4$ is hydrogen, $-NH_2$ or lower alkyl;
$-NH_2$; $-CH_2NH_2$; $-CH_2ONH_2$; $-CH_2ON=CH_2$;
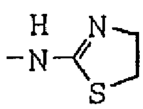 ; 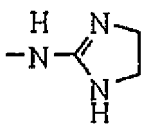 ; 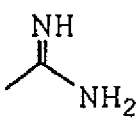 ; 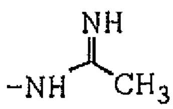 or 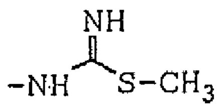 .

[4] The composition of [3], wherein, in the formula (II), $R^2$ is the formula:
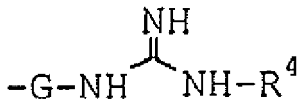

wherein G is a bond, $-NHCOCH_2-$ or lower alkylene, and $R^4$ is hydrogen, $-NH_2$ or lower alkyl; $-NH_2$; $-CH_2NH_2$; $-CH_2ONH_2$; $-CH_2ON=CH_2$;

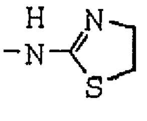 ; 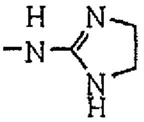 ; 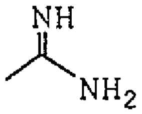 ; 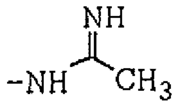 or 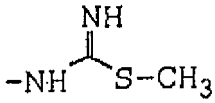 .

[5] The composition of [4], wherein, in the formula (I), $R^2$ is the following formula (III):

J-L-M (III)

wherein J is $-NR^{2a}-$, $-NR^{2a}-CO-$, $-(CH_2)_n-$ or $-(CH_2)_nCO-$ wherein $R^{2a}$ is hydrogen, lower alkyl, or acyl; n is an integer of 0 to 6;

L is $-NR^{2b}-$ wherein $R^{2b}$ is hydrogen, lower alkyl, alkoxycarbonyl or acyl; and

M is optionally substituted amino.

[6] The composition of [5], wherein, in the formula (III),. J-L-M is $-CO-NH-NH_2$, $-CH_2-CO-NH-NH_2$, $-CH_2-CO-NH-NH-CH_3$, $-CH_2-CO-N(CH_3)-NH_2$, $-CH_2-CO-NH-NH-C_2H_5$, $-CH_2-CO-NH-N(CH_3)_2$, $-(CH_2)_2-CO-NH-NH_2$, $-NH-CO-NH-NH_2$, $-NH-NH_2$, $-CH_2-NH-NH_2$, $-(CH_2)_2-NH-NH_2$ or $-(CH_2)_3-NH-NH_2$.

[7] The composition of any of [2] - [6], wherein, in the formula (I), $R^1$ is alkylcarbonyl, and X is a divalent residue induced from thiazole optionally substituted by methylsulfonylbenzyl.

[8] The composition of [1], wherein the aforementioned VAP-1 inhibitor is N-{4-[2-(4-hydrazinocarbonylmethylphenyl) ethyl]-1,3-thiazol-2-yl}acetamide;

or a derivative thereof;

or a pharmaceutically acceptable salt thereof.

[9] The composition of [1], wherein the aforementioned VAP-1 inhibitor is

N-(4-{2-[4-(2-{[amino(imino)methyl]amino}ethyl)phenyl]ethyl}-1,3-thiazol-2-yl)acetamide;

or a derivative thereof;

or a pharmaceutically acceptable salt thereof.

[10] The composition of any of [1] - [9], wherein the aforementioned ophthalmic disease is ischemic retinopathy or ischemic optic neuropathy.

[11] The composition of any of [1] - [9], wherein the aforementioned ophthalmic disease is retinopathy of prematurity, proliferative diabetic retinopathy, polypoid choroidal vasculopathy, retinal angiomatous proliferation, retinal artery occlusion, retinal vein occlusion, Coats' disease, familial exudative vitreoretinopathy, pulseless disease (Takayasu disease), Eales' disease, antiphospholipid syndrome, leukemiaretinopathy, blood hyperviscosity syndrome, macroglobulinemia, interferon retinopathy, hypertensive retinopathy, radiation retinopathy or cornea epithelial stem cell deficiency.

[12] The composition of [11], wherein the aforementioned ophthalmic disease is retinopathy of prematurity.

[13] Use of a VAP-1 inhibitor for the production of a medicament for the treatment of an ophthalmic disease associated with hypoxia or ischemia.

[14] A method of treating an ophthalmic disease associated with hypoxia or ischemia, comprising a step of administering, to a subject in need of the treatment, a VAP-1 inhibitor in an amount sufficient to treat the subject for the disease.

[15] A commercial package comprising the composition of any of [1] - [12], and a written matter stating that the composition can or should be used for treating an ophthalmic disease associated with hypoxia or ischemia.

[16] An angiogenesis inhibitor composition comprising a VAP-1 inhibitor as an active ingredient.

[17] The composition of [16], wherein the aforementioned VAP-1 inhibitor is a compound represented by the following formula (I):

$R^1$-NH-X-Y-Z (I)

wherein

$R^1$ is acyl;
X is a divalent residue induced from optionally substituted thiazole;
Y is the formula: $Y^1$-$Y^2$-$Y^3$
wherein $Y^1$ is a bond, lower alkylene, lower alkenylene, lower alkynylene, $-(CH_2)_n$-O-, $-(CH_2)_n$-NH-, $-(CH_2)_n$-CO- or -$(CH_2)_n$-$SO_2$- (wherein n is an integer of 0 to 6);
$Y^2$ is a bond, -O-, -NH-, -CO- or -$SO_2$-;
$Y^3$ is a bond, lower alkylene, lower alkenylene or lower alkynylene, provided that when $Y^1$ is $-(CH_2)_n$-O-, then $Y^2$ is not -O-, -NH- or -$SO_2$- when $Y^1$ is $-(CH_2)_n$-NH-, then $Y^2$ is not -O- or -NH-, when $Y^1$ is $-(CH_2)_n$-CO-, then $Y^2$ is not -CO-, and when $Y^1$ is $-(CH_2)_n$-$SO_2$-, then $Y^2$ is not -O-, and -$SO_2$-(wherein n is as defined above);
Z is the formula:
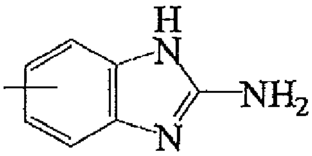
,
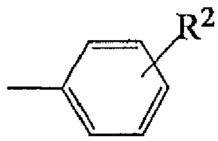
or
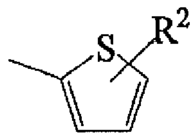

wherein $R^2$ is the formula: -A-B-D-E
wherein
A is a bond, lower alkylene, -$NR^{2a}$- or -$SO_2$- wherein $R^{2a}$ is hydrogen, lower alkyl or acyl;
B is a bond, lower alkylene, -CO- or -O-;
D is a bond, lower alkylene, -$NR^{2b}$- or -$CH_2NH$- wherein $R^{2b}$ is hydrogen, lower alkyl, alkoxycarbonyl or acyl; and E is optionally substituted amino, -N=$CH_2$,
**[0026]**

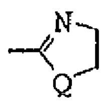
or
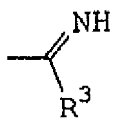

wherein
Q is -S- or -NH-;
$R^3$ is hydrogen, lower alkyl, lower alkylthio or -NH-$R^4$
wherein $R^4$ is hydrogen, -$NH_2$ or lower alkyl,
or a derivative thereof, or a pharmaceutically acceptable salt thereof.
[18] The composition of [17], wherein, in the formula (I), Z is the formula (II):
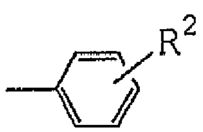
(II)

wherein
$R^2$ is the formula:
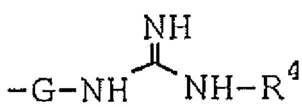

wherein G is a bond, -$NHCOCH_2$- or lower alkylene; $R^4$ is hydrogen, -$NH_2$ or lower alkyl;
-$NH_2$; -$CH_2NH_2$; -$CH_2ONH_2$; -$CH_2ON$=$CH_2$;

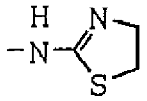 ; 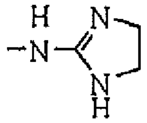 ; 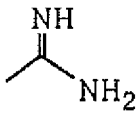 ; 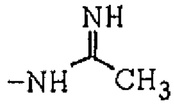 or 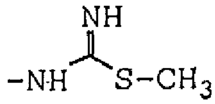 .

[19] The composition of [18], wherein, in the formula (II), $R^2$ is the formula:

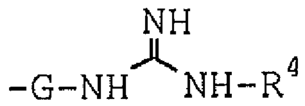

wherein G is a bond, $-NHCOCH_2-$ or lower alkylene, and $R^4$ is hydrogen, $-NH_2$ or lower alkyl; $-NH_2$; $-CH_2NH_2$; $-CH_2ONH_2$; $-CH_2ON=CH_2$;

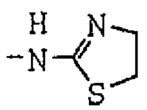 ; 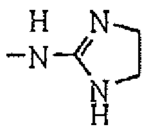 ; 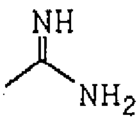 ; 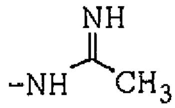 or 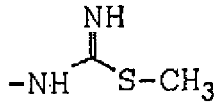 .

[20] The composition of [19], wherein, in the formula (I), $R^2$ is the following formula (III):

J-L-M (III)

wherein
J is $-NR^{2a}-$, $-NR^{2a}-CO-$, $-(CH_2)_n-$ or $-(CH_2)_nCO-$ wherein $R^{2a}$ is hydrogen, lower alkyl or acyl; and n is an integer of 0 to 6;
L is $-NR^{2b}-$ wherein $R^{2b}$ is hydrogen, lower alkyl, alkoxycarbonyl or acyl; and
M is optionally substituted amino.

[21] The composition of [20], wherein, in the formula (III),
J-L-M is
$-CO-NH-NH_2$, $-CH_2-CO-NH-NH_2$, $-CH_2-CO-NH-NH-CH_3$, $-CH_2-CO-N(CH_3)-NH_2$, $-CH_2-CO-NH-NH-C_2H_5$, $-CH_2-CO-NH-N(CH_3)_2$, $-(CH_2)_2-CO-NH-NH_2$, $-NH-CO-NH-NH_2$, $-NH-NH_2$, $-CH_2-NH-NH_2$, $-(CH_2)_2-NH-NH_2$ or $-(CH_2)_3-NH-NH_2$.

[22] The composition of any of [17] - [21], wherein, in the formula (I), $R^1$ is alkylcarbonyl, and X is a divalent residue induced from thiazole optionally substituted by methylsulfonylbenzyl.

[23] The composition of [16], wherein the aforementioned VAP-1 inhibitor is N-{4-[2-(4-hydrazinocarbonylmethylphenyl) ethyl]-1,3-thiazol-2-yl)acetamide;
or a derivative thereof;
or a pharmaceutically acceptable salt thereof.

[24] The composition of [16], wherein the aforementioned VAP-1 inhibitor is
N-(4-{2-[4-(2-{[amino(imino)methyl]amino}ethyl)phenyl]ethyl}-1,3-thiazol-2-yl)acetamide;
or a derivative thereof;
or a pharmaceutically acceptable salt thereof.

[25] The composition of any of [16] - [24], wherein the blood vessel is an ocular blood vessel.

[26] Use of a VAP-1 inhibitor for the production of a medicament for the suppression of angiogenesis.

[27] A method of suppressing angiogenesis, comprising a step of administering, to a subject in need of a treatment, a VAP-1 inhibitor in an amount sufficient to suppress angiogenesis in the subject.

[28] A commercial package comprising the composition of any of [16] - [25], and a written matter stating that the composition can or should be used for treating a disease requiring suppression of angiogenesis.

Effect of the Invention

The composition of the present invention comprising a VAP-1 inhibitor as an active ingredient can be used for the prophylaxis or treatment of ophthalmic diseases associated with hypoxia or ischemia. The angiogenesis inhibitor composition of the present invention comprising a VAP-1 inhibitor as an active ingredient can suppress pathologic angiogenesis.

Best Mode for Carrying out the Invention

The present invention provides a composition for ophthalmic diseases associated with hypoxia or ischemia, which contains a VAP-1 (vascular adhesion protein-1) inhibitor as an active ingredient.
The hypoxia in the present invention refers to a condition where oxygen is depleted in the biological tissues, and the ischemia refers to a condition where the blood is not supplied due to, for example, stenosis or complete obstruction of the intravascular lumen.
Examples of the ophthalmic diseases associated with hypoxia or ischemia in the present invention include ischemic retinopathy, ischemic optic neuropathy and the like.
In addition, examples of the ophthalmic diseases associated with hypoxia or ischemia in the present invention include retinopathy of prematurity, polypoid choroidal vasculopathy, retinal angiomatous proliferation, retinal artery occlusion, retinal vein occlusion, Coats' disease, familial exudative vitreoretinopathy, pulseless disease (Takayasu disease), Eales' disease, antiphospholipid syndrome, leukemiaretinopathy, blood hyperviscosity syndrome, macroglobulinemia, interferon retinopathy, hypertensive retinopathy, radiation retinopathy, cornea epithelial stem cell deficiency and the like, particularly, retinopathy of prematurity.
The diabetic retinopathy is classified into non-proliferative diabetic retinopathy and proliferative diabetic retinopathy, and the non-proliferative type and proliferative type are known to differ in the expression mechanism. The non-proliferative type, which is free of neovessel, is caused by metabolic disorder or inflammation, and accompanies intraretinal hemorrhage, leukoderma, macular edema and the like. On the other hand, the proliferative type is caused by ischemia, and accompanies neovessel, vitreous hemorrhage and the like. Thus, proliferative diabetic retinopathy can also be recited as an ophthalmic disease associated with hypoxia or ischemia in the present invention.
In addition, the present invention provides an angiogenesis inhibitor composition. The angiogenesis inhibitor composition of the present invention contains a VAP-1 inhibitor as an active ingredient. Angiogenesis occurs as a physiological phenomenon during growth, development, follicle formation, wound healing, pregnancy and the like. On the other hand, it is also observed in a diseased condition such as inflammation, growth of solid tumor, diabetic retinopathy and the like. The angiogenesis inhibitor composition of the present invention suppresses angiogenesis in a diseased condition. Specifically, it suppresses angiogenesis developed in hypoxia or ischemia conditions. The angiogenesis inhibitor composition of the present invention suppresses angiogenesis of ocular blood vessel, preferably retina blood vessel.
Examples of the VAP-1 inhibitor as the active ingredient of the composition of the present invention include a compound represented by the following formula (I), a derivative thereof, and a pharmaceutically acceptable salt thereof.
Formula (I):
$$R^1\text{-NH-X-Y-Z} \qquad (I)$$

wherein
$R^1$ is acyl;
X is a divalent residue derived from optionally substituted thiazole;
Y is the formula: $Y^1$-$Y^2$-$Y^3$
wherein $Y^1$ is a bond, lower alkylene, lower alkenylene, lower alkynylene, $-(CH_2)_n$-O-, $-(CH_2)_n$-NH-, $-(CH_2)_n$-CO- or $-(CH_2)_n-SO_2-$ (wherein n is an integer of 0 to 6);
$Y^2$ is a bond, -O-, -NH-, -CO- or $-SO_2-$;
$Y^3$ is a bond, lower alkylene, lower alkenylene or lower alkynylene, provided that when $Y^1$ is $-(CHz)_n$-O-, then $Y^2$ is not -O-, -NH- or $-SO_2-$, when $Y^1$ is $-(CH_2)_n$-NH-, then $Y^2$ is not -O- or -NH-, when $Y^1$ is $-(CH_2)_n$-CO-, then $Y^2$ is not -CO-, when $Y^1$ is $-(CH_2)_n-SO_2-$, then $Y^2$ is not -O- and $-SO_2-$ (wherein n is as defined above);
Z is the formula:
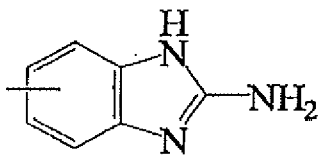 , 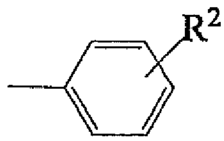 or 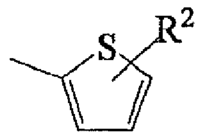

wherein $R^2$ is the formula: -A-B-D-E
wherein
A is a bond, lower alkylene, $-NR^{2a}-$ or $-SO_2-$ wherein $R^{2a}$ is hydrogen, lower alkyl or acyl;
B is a bond, lower alkylene, -CO- or -O-;
D is a bond, lower alkylene, $-NR^{2b}-$ or $-CH_2NH-$ wherein $R^{2b}$ is hydrogen, lower alkyl, alkoxycarbonyl or acyl;
E is optionally substituted amino, $-N=CH_2$,

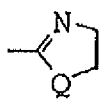 or 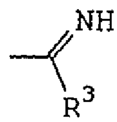

wherein Q is -S- or -NH-; $R^3$ is hydrogen, lower alkyl, lower alkylthio or $-NH-R^4$ wherein $R^4$ is hydrogen, $-NH_2$ or lower alkyl.

The terms used for the present invention in the above- and below-mentioned descriptions of the present specification are explained in detail in the following.

The term "lower" is used to mean a group having a carbon number of 1 to 6, preferably 1 to 4, unless otherwise specified.

Examples of the "lower alkyl" include a straight chain or branched chain alkyl having a carbon number of 1 to 6 (e.g., methyl, ethyl, propyl, isopropyl, butyl, isobutyl, sec-butyl, tert-butyl, pentyl, tert-pentyl and hexyl) and the like. Among these, $C_1$-$C_4$ alkyl is more preferable.

Examples of the "lower alkylene" include a straight chain or branched chain alkylene having a carbon number of 1 to 6 (e.g., methylene, ethylene, trimethylene, tetramethylene, propylene, ethylidene and propylidene) and the like. Among these, $C_1$-$C_4$ alkylene is more preferable.

Examples of the "lower alkenylene" include a straight chain or branched chain alkenylene having a carbon number of 2 to 6 (e.g., vinylene, 1-propenylene, 2-propenylene, 1-butenylene, 2-butenylene, 3-butenylene, 1,3-butadienylene, 1-methyl-1-propenylene, 2-methyl-1-propenylene, 1-pentenylene, 2-pentenylene, 3-pentenylene, 4-pentenylene, 1,3-pentadienylene, 2-penten-4-ynylene, 3,3-dimethyl-1-propenylene, 2-ethyl-1-propenylene, 1-hexenylene, 2-hexenylene, 3-hexenylene, 4-hexenylene, 5-hexenylene, 1,3-hexadienylene, 1,4-hexadienylene, 1,3,5-hexatrienylene) and the like. Of these, $C_2$-$C_4$ alkenylene is more preferable.
The above-mentioned lower alkenylene may be an E-form or Z-form. When the compound of the present invention has a lower alkenylene moiety, the compound of the present invention encompasses any stereoisomer wherein the lower alkenylene moiety is an E-structure or Z-structure.

Examples of the "lower alkynylene" include a straight chain or branched chain alkynylene having a carbon number of 2 to 6, which has 1 to 3 triple bonds (e.g., ethynylene, 1-propynylene, 2-propynylene, 1-butynylene, 2-butynylene, 3-butynylene, 1-pentynylene, 2-pentynylene, 3-pentynylene, 4-pentynylene, 2-pentyn-4-ynylene, 1-hexynylene, 2-hexynylene, 3-hexynylene, 4-hexynylene, 5-hexynylene, 3-ethyl-1-propynylene, 3,3-diethyl-1-propynylene). Of these, $C_2$-$C_4$ alkynylene is more preferable.

The "lower alkylthio" is a group wherein a sulfur atom is bonded to the alkyl moiety, which is straight chain or branched chain, the above-mentioned lower alkyl group having a carbon number of 1 to 6, and examples thereof include methylthio, ethylthio, propylthio, isopropylthio, butylthio, isobutylthio, sec-butylthio, tert-butylthio, pentylthio, tert-pentylthio, hexylthio and the like.

Examples of the "alkylcarbonyl" include alkylcarbonyl wherein the alkyl moiety has a carbon number of 1 to 6 [that is, the alkyl moiety is $C_1$-$C_6$ alkyl of the above-mentioned "lower alkyl"] (e.g., acetyl, propionyl, butyryl, isobutyryl, valeryl, isovaleryl, pivaloyl, hexanoyl and heptanoyl) and the like.

Examples of the "aryl" include $C_6$-$C_{10}$ aryl (e.g., phenyl and naphthyl) and the like, where the "aryl" may be substituted by 1 to 3 substituents and the position of substitution is not particularly limited. Examples of the substituent include halogen, lower alkyl, halogenated lower alkyl, lower alkoxy, lower acyl and the like.

Examples of the "aralkyl" include aralkyl wherein the aryl moiety has a carbon number of 6 to 10 [that is, the aryl moiety is $C_6$-$C_{10}$ aryl of the above-mentioned "aryl"], and the alkyl moiety has a carbon number of 1 to 6 [that is,

the alkyl moiety is $C_1$-$C_6$ alkyl of the above-mentioned "lower alkyl"] (e.g., benzyl, phenethyl, 1-naphthylmethyl, 2-naphthylmethyl, 3-phenylpropyl, 4-phenylbutyl and 5-phenylpentyl) and the like.

The "amino" of the "optionally substituted amino" may be substituted by 1 or 2 substituents, and the substituent may be a protecting group. That is, the "optionally substituted amino" is represented by the formula $-NR^{5a}R^{5b}$.

Examples of $R^{5a}$ or $R^{5b}$ include lower alkyl, acyl, alkoxycarbonyl, aryl, aralkyl, cyclo(lower)alkyl, cyclo(lower) alkoxycarbonyl, sulfuryl, sulfinyl, phosphoryl, heterocyclic group and the like, each of which is unsubstituted or optionally substituted, and hydrogen. The lower alkyl, acyl, alkoxycarbonyl, aryl and aralkyl are as defined above or below. Examples of cyclo(lower)alkyl include cycloalkyl having a carbon atom and having a carbon number of 3 to 6 (e.g., cyclopropyl, cyclobutyl, cyclopentyl, cyclohexyl). Examples of cyclo(lower)alkoxycarbonyl include cycloalkoxycarbonyl wherein the cycloalkyl moiety has a carbon atom and having a carbon number of 3 to 6 (e.g., cyclopropyloxycarbonyl, cyclobutyloxycarbonyl, cyclopentyloxycarbonyl, cyclohexyloxycarbonyl). In addition, they may be protected according to the method described in "Protective Groups in Organic Synthesis 3rd Edition" (published by John Wiley and Sons, 1999) and the like. $R^{5a}$ and $R^{5b}$ may be the same or different.

Examples of the "heterocycle" include "aromatic heterocycle" and "non-aromatic heterocycle". Examples of the "aromatic heterocycle" include a 5- to 10-membered aromatic heterocycle containing, besides carbon atoms, 1 to 3 hetero atoms selected from nitrogen, oxygen and sulfur atom and the like, for example, thiophene, furan, pyrrole, imidazole, pyrazole, thiazole, isothiazole, oxazole, isoxazole, pyridine, pyridazine, pyrimidine, pyrazine and the like. Examples of the "non-aromatic heterocycle" include a 5- to 10-membered non-aromatic heterocycle containing, besides carbon atoms, 1 to 3 hetero atom selected from nitrogen, oxygen and sulfur atom and the like, for example, pyrrolidine, imidazoline, pyrazolidine, pyrazoline, piperidine, piperazine, morpholine, thiomorpholine, dioxolane, oxazolidine, thiazolidine, triazolysine and the like.

Examples of the "acyl" include alkylcarbonyl, arylcarbonyl and the like.

Examples of the "alkylcarbonyl" include alkylcarbonyl wherein the alkyl moiety has 1 to 6 carbon atoms [that is, the alkyl moiety is $C_1$-$C_6$ alkyl of the above-mentioned "lower alkyl"] (e.g., acetyl, propionyl, butyryl, isobutyryl, valeryl, isovaleryl, pivaloyl, hexanoyl and heptanoyl) and the like.

Examples of the "arylcarbonyl" include arylcarbonyl wherein the aryl moiety has 6 to 10 carbon atoms [that is, the aryl moiety is $C_6$-$C_{10}$ aryl of the above-mentioned "aryl"] (e.g., benzoyl and naphthoyl) and the like.

Examples of the "alkoxycarbonyl" include alkyloxycarbonyl, aralkyloxycarbonyl and the like.

Examples of the "alkyloxycarbonyl" include alkyloxycarbonyl wherein the alkyl moiety has a carbon number of 1 to 10 (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, tert-pentyloxycarbonyl, hexyloxycarbonyl etc.) and the like.

Examples of the "aralkyloxycarbonyl" include aralkyloxycarbonyl wherein the aryl moiety has a carbon number of 6 to 10 [that is, the aryl moiety is $C_6$-$C_{10}$ aryl of the above-mentioned "aryl"], and the alkyl moiety has a carbon number of 1 to 6 [that is, the alkyl moiety is $C_1$-$C_6$ alkyl of the above-mentioned "lower alkyl"] (e.g., benzyloxycarbonyl, phenethyloxycarbonyl, 1-naphthylmethyloxycarbonyl, 2-naphthylmethyloxycarbonyl, 3-phenylpropyloxycarbonyl, 4-phenylbutyloxycarbonyl and 5-phenylpentyloxycarbonyl etc.) and the like.

Examples of the "divalent residue derived from the optionally substituted thiazole" include

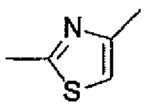 and 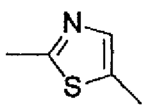

The "thiazole" may have a substituent, and the position of substitution is not particularly limited. Examples of the "substituent" of the above-mentioned "optionally substituted thiazole" include a group described in the following (1) - (12) and the like.

(1) halogen (e.g., fluorine, chlorine, bromine);

(2) alkoxycarbonyl defined above (e.g., ethoxycarbonyl);

(3) optionally substituted aryl (said aryl is as defined above, and may be substituted by $-SO_2$-(lower alkyl) (wherein the lower alkyl is as defined above and the like) at any substitutable position not particularly limited (e.g., phenyl and 4-(methylsulfonyl)phenyl);

(4) a group of the formula: $-CONR^aR^b$ wherein $R^a$ is hydrogen, lower alkyl, aryl or aralkyl, $R^b$ is hydrogen, lower alkyl, aryl or aralkyl, where the lower alkyl, aryl and aralkyl are as defined above (e.g., N-methylaminocarbonyl, N-phenylaminocarbonyl, N,N-dimethylaminocarbonyl and N-benzylaminocarbonyl);

(5) a group of the formula: $-CONH-(CH_2)_k$-aryl wherein k is an integer of 0 to 6; aryl is as defined above, optionally has 1 to 5 substituents selected from the group consisting of $-NO_2$, $-SO_2$-(lower alkyl) wherein the lower alkyl

is as defined above, $-CF_3$ and -O-aryl wherein aryl is as defined above, where the position of substitution is not particularly limited;

(6) a group of the formula: $-CONH-(CH_2)_m$-heterocycle wherein m is an integer of 0 to 6; and heterocycle is as defined above (e.g., pyridine);

(7) a group of the formula: -CO-heterocycle wherein heterocycle is as defined above (e.g., pyrrolidine, piperidine, piperazine, thiomorpholine), and heterocycle optionally has 1 to 5 substituents selected from the group consisting of -CO-(lower alkyl) wherein the lower alkyl is as defined above, - CO-O-(lower alkyl) wherein the lower alkyl is as defined above, $-SO_2$-(lower alkyl) wherein the lower alkyl is as defined above, oxo (i.e., =O) and a group of the formula: $-CONR^cR^d$ wherein $R^c$ is hydrogen, lower alkyl, aryl or aralkyl, $R^d$ is hydrogen, lower alkyl, aryl or aralkyl, and lower alkyl, aryl and aralkyl are as defined above, where the position of substitution is not particularly limited;

(8) a group of the formula: $-(CH_2)_t$-aryl wherein t is an integer of 1 to 6; aryl is as defined above, and optionally has 1 to 5 substituents selected from the group consisting of -S-(lower alkyl) wherein lower alkyl is as defined above, $-SO_2$-(lower alkyl) wherein lower alkyl is as defined above, $-SO_2-NR^vR^w$ wherein $R^v$ is hydrogen, lower alkyl, aryl or aralkyl, $R^w$ is hydrogen, lower alkyl, aryl or aralkyl, and lower alkyl, aryl and aralkyl are as defined above, $-CO_2$-(lower alkyl) wherein lower alkyl is as defined above, -NHCO-O-(lower alkyl) wherein lower alkyl is as defined above and a group of the formula: $-CONR^eR^f$ wherein $R^e$ is hydrogen, lower alkyl, aryl or aralkyl, $R^f$ is hydrogen, lower alkyl, aryl or aralkyl, and lower alkyl, aryl and aralkyl are as defined above, where the position of substitution is not particularly limited;

(9) a group of the formula: $-(CH^2)_o$-heterocycle wherein o is an integer of 0 to 6; heterocycle is as defined above (e.g., pyrrolidine, piperidine, piperazine, morpholine, thiomorpholine), and optionally has 1 to 5 substituents selected from the group consisting of oxo (that is, =O); -CO-(lower alkyl) wherein lower alkyl is as defined above; -CO-O-(lower alkyl) wherein lower alkyl is as defined above; $-SO_2$-(lower alkyl) wherein lower alkyl is as defined above; -CO-(heterocycle) wherein heterocycle is as defined above (e.g., pyrrolidine, piperazine and morpholine), and optionally has 1 to 5 substituents selected from the group consisting of lower alkyl (lower alkyl is as defined above) and halogen (e.g., fluorine, chlorine, bromine), where the position of substitution is not particularly limited; and a group of the formula: $-CONR^gR^h$ wherein $R^g$ is hydrogen, lower alkyl, aryl or aralkyl, $R^h$ is hydrogen, lower alkyl, aryl or aralkyl, and lower alkyl, aryl and aralkyl are as defined above, where the position of substitution is not particularly limited;

(10) a group of the formula: $-(CH_2)_p-NR^iR^j$ wherein p is an integer of 0 - 6; $R^i$ is hydrogen, acyl, lower alkyl, aryl or aralkyl, $R^j$ is hydrogen, acyl, lower alkyl, aryl or aralkyl, and acyl, lower alkyl, aryl and aralkyl are as defined above, and lower alkyl optionally has 1 to 5 substituents selected from the group consisting of a group of the formula: $-CONR^kR^l$ wherein $R^k$ is hydrogen, lower alkyl, aryl or aralkyl, $R^l$ is hydrogen, lower alkyl, aryl or aralkyl, and lower alkyl, aryl and aralkyl are as defined above, where the position of substitution is not particularly limited;

(11) a group of the formula: -CON(H or lower alkyl)-$(CHR^m)_q$-T wherein q is an integer of 0 to 6; lower alkyl is as defined above; $R^m$ is hydrogen, aralkyl defined above or alkyl defined above (particularly lower alkyl), these are optionally substituted by 1 to 3 substituents selected from the group consisting of -OH and $-CONH_2$, where the position of substitution is not particularly limited; T is hydrogen; a group of the formula: $-CONR^nR^o$ wherein $R^n$ is hydrogen, lower alkyl, aryl or aralkyl, $R^o$ is hydrogen, lower alkyl, aryl or aralkyl, and lower alkyl, aryl and aralkyl are as defined above; $-NH-CO-R^p$ group wherein $R^p$ is lower alkyl defined above or aralkyl defined above; $-NH-SO_2$-(lower alkyl) group wherein lower alkyl is as defined above; $-SO_2$-(lower alkyl) group wherein lower alkyl is as defined above; -heterocycle group wherein heterocycle is as defined above (e.g., pyridine, pyrrolidine and morpholine), optionally has 1 to 3 substituents (e.g., oxo (that is, =O)), where the position of substitution is not particularly limited; or -CO-(heterocycle) group wherein heterocycle is as defined above (e.g., piperidine and morpholine); and

(12) a group of the formula: $-(CH_2)_r-CO-NR^tR^u$ wherein r is an integer of 1 to 6; $R^t$ is hydrogen, lower alkyl, aryl or aralkyl, $R^u$ is hydrogen, lower alkyl, aryl or aralkyl, and lower alkyl, aryl and aralkyl are as defined above.

The position of substitution on aryl or heterocycle may be any and is not particularly limited. Preferable "substituent" of the above-mentioned "optionally substituted thiazole" is methylsulfonylbenzyl, sulfamoylbenzyl (e.g., 4-sulfamoylbenzyl) and the like. The position of substitution of the methylsulfonyl group, sulfamoyl group and the like is not particularly limited.

Preferable example of $R^1$ is alkylcarbonyl (which is as defined above), and more preferable example is acetyl. As the "divalent residue derived from thiazole" moiety of the "divalent residue derived from optionally substituted thiazole" for X,

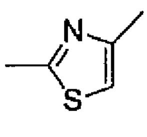

is preferable. As the "substituent" of the "divalent residue derived from optionally substituted thiazole", methylsulfonylbenzyl, sulfamoylbenzyl (e.g., 4-sulfamoylbenzyl) and the like are preferable.

Lower alkylene, lower alkenylene and lower alkynylene for $Y^1$ or $Y^3$ of the formula: $Y^1$-$Y^2$-$Y^3$ for Y in the formula (I) may be those defined above and the like.

Specific examples of the formula: $Y^1$-$Y^2$-$Y^3$ for Y in the formula (I) include $-(CH_2)_{n'}-$ wherein n' is an integer of 1 to 6, $-(CH_2)_n-NH-(CH_2)_n-$, $-(CH_2)_n-O-(CH_2)_n-$, $-(CH_2)_n-CO-O-(CH_2)_n-$, $-(CH_2)_n-O-CO-(CH_2)_n-$, $-(CH_2)_n-CO-NH-(CH_2)_n-$, $-(CH_2)_n-NH-CO-(CH_2)_n-$, $-(CH_2)_n-SO_2-NH-(CH_2)_n-$ and $-(CH_2)_n-NH-SO_2-(CH_2)_n-$ (in each of which n is an integer of 0 to 6) and the like. Of these, $-(CH_2)_{n'}-$, $-(CH_2)_n-NH-(CH_2)_n-$, $-(CH_2)_n-CO-O-(CH_2)_n-$ or $-(CH_2)_n-CO-NH-(CH_2)_n-$ is preferable, and $-(CH_2)_n-$ is particularly preferable. Specific examples include $-(CH_2)_2-$, $-CH_2-CO-$, $-CH_2-NH-$, -CO-O-, -CO-NH- and the like.

A preferable example of Z is the following formula (II):

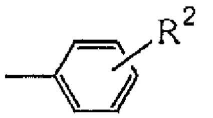

(I I)

wherein $R^2$ is a group of the following formula:

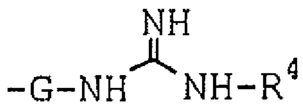

wherein G is a bond, $-NHCOCH_2-$ or lower alkylene (as defined above), and $R^4$ is hydrogen, $-NH_2$ or lower alkyl (as defined above);

$-NH_2$; $-CH_2NH_2$; $-CH_2ONH_2$; $-CH_2ON=CH_2$;

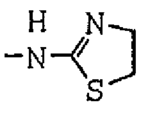 ; 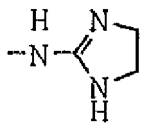 ; 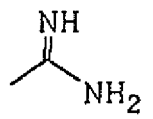 ; 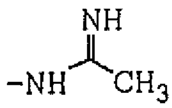 ; 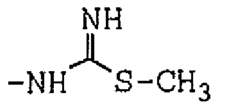 ;

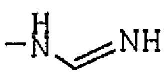 or 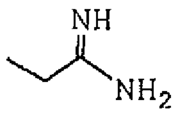 .

More preferably, $R^2$ is a group of the following formula:

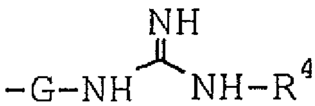

wherein G is a bond, $-NHCOCH_2-$ or lower alkylene (as defined above), and $R^4$ is hydrogen, $-NH_2$ or lower alkyl (as defined above);

$-NH_2$; $-CH_2NH_2$; $-CH_2ONH_2$; $-CH_2ON=CH_2$;

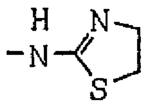 ; 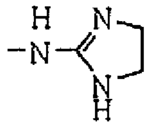 ; 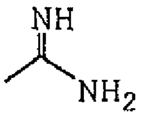 ; 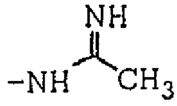 or 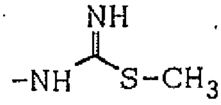

In addition, a preferable example of $R^2$ is the following formula (III):

J-L-M (III)

wherein J is $-NR^{2a}-$, $-NR^{2a}-CO-$, $-(CH_2)_n-$ or $-(CH_2)_nCO-$ (wherein $R^{2a}$ is hydrogen, lower alkyl (as defined above), or acyl (as defined above); n is an integer of 0 to 6);

L is $-NR^{2b}-$ wherein $R^{2b}$ is hydrogen, lower alkyl (as defined above), alkoxycarbonyl (as defined above) or acyl (as defined above); and

M is optionally substituted amino.

Furthermore, a preferable example of the J-L-M moiety (molecular terminal) is a group wherein J is a bond, -NH-CO- or $-(CH_2)_nCO-$ wherein n is an integer of 0 to 2;

L is -NH- or $-N(CH_3)-$; and,

M is optionally substituted amino.

Specific examples of the J-L-M moiety include $-CO-NH-NH_2$, $-CH_2-CO-NH-NH_2$, $-CH_2-CO-NH-NH-CH_3$, $-CH_2-CO-N(CH_3)-NH_2$, $-CH_2-CO-NH-NH-C_2H_5$, $-CH_2-CO-NH-N(CH_{3)2}$, $-(CH_2)_2-CO-NH-NH_2$, $-NH-CO-NH-NH_2$, $-NH-NH_2$, $-CH_2-NH-NH_2$, $-(CH_2)_2-NH-NH_2$, $-(CH_2)_3-NH-NH_2$ and the like.

In the formula (I) in the present invention, $R^1$ is preferably alkylcarbonyl, and X is preferably a divalent residue derived from thiazole optionally substituted by methylsulfonylbenzyl.

Moreover, the compound represented by the formula (I) in the present invention is preferably N-{4-[2-(4-hydrazinocarbonylmethylphenyl)ethyl]-1,3-thiazol-2-yl}acetamide, N-(4-{2-[4-(2-([amino(imino)methyl]amino)ethyl)phenyl]ethyl}-1,3-thiazol-2-yl)acetamide or the like.

When the compound represented by the formula (I) in the present invention has an asymmetric carbon atom in the structure, the invention encompasses all enantiomers and diastereomers. In the present invention, moreover, a derivative of the compound represented by the formula (I) can also be used. Such derivative is not particularly limited as long as it can exhibit the action of the present invention.

The VAP-1 inhibitor and a derivative thereof of the present invention can also be converted to a pharmaceutically acceptable salt. The pharmaceutically acceptable salt in the present invention is not particularly limited as long as it is a nontoxic pharmaceutically acceptable general salt, and a salt with an inorganic or organic base, acid addition salt and the like can be mentioned. Examples of the salt with an inorganic or organic base include alkali metal salt (e.g., sodium salt, potassium salt etc.), alkaline earth metal salt (e.g., calcium salt, magnesium salt etc.), ammonium salt, and amine salt (e.g., triethylamine salt, N-benzyl-N-methylamine salt etc.) and the like. Examples of the acid addition salt include salts derived from mineral acid (e.g., hydrochloric acid, hydrobromic acid, hydroiodic acid, phosphoric acid, metaphosphoric acid, nitric acid and sulfuric acid), and salts derived from organic acid (e.g., tartaric acid, acetic acid, citric acid, malic acid, lactic acid, fumaric acid, maleic acid, benzoic acid, glycol acid, gluconic acid, succinic acid and arylsulfonic acid (e.g., p-toluenesulfonic acid)) and the like.

The compound represented by the formula (I) and a derivative thereof, and a pharmaceutically acceptable salt thereof of the present invention can be used as medicaments and the like in the form of a prodrug. The term "prodrug" means any compounds that can be converted to a VAP-1 inhibitor in the body after administration. The prodrug may be any pharmaceutically acceptable prodrug of a composition for ophthalmic diseases associated with hypoxia or ischemia or angiogenesis inhibitor of the present invention.

In the following explanation, unless otherwise specified, both the "composition for ophthalmic diseases associated with hypoxia or ischemia" and the "angiogenesis inhibitor composition" are generically referred to as the "composition of the present invention".

The composition of the present invention can be administered by any route. Examples of the administration route include systemic administration (e.g., oral administration or injection administration), topical administration (e.g., ocular instillation, eye ointment), periocular administration (e.g., sub-Tenon's capsule administration), conjunctiva administration, intraocular administration, subretinal administration, suprachoroidal administration, retrobulbar administration and the like. The administration route of the composition of the present invention can be appropriately determined according to whether the application to an ophthalmic disease associated with hypoxia or ischemia aims at prophylaxis or treatment and the like. Preferable administration route is an ophthalmic topical administration.

The composition of the present invention is preferably administered rapidly to an administration subject such

as mammal, particularly human, after diagnosis of the risk of ophthalmic disease associated with hypoxia or ischemia, or pathologic angiogenesis, before the onset thereof
(prophylactic treatment). Alternatively, it is rapidly administered to the administration subject after the onset of an ophthalmic disease associated with hypoxia or ischemia or pathologic angiogenesis (therapeutic treatment). The treatment plan can be appropriately determined according to the kind of the active ingredient to be used, dose, administration route, cause and, where necessary, the level of subjective symptoms of an ophthalmic disease associated with hypoxia or ischemia and the like.

As an administration method of the composition of the present invention, a method known per se for general medicaments can be used. The administration route may be an appropriately effective one and one or more routes can be used. Accordingly, the above-mentioned administration routes are mere exemplifications free of any limitation.

The dosage (dose) of the composition of the present invention for a subject of administration such as animal including human, particularly human, is an amount sufficient to provide a desired response in the subject of administration for a reasonable period of time. The dose is appropriately determined according to various factors including the strength of the active ingredient to be used, age, species, symptom, disease state, body weight and severity of disease of the subject of administration, the route, timing and frequency of the administration and the like. The dose can also be appropriately controlled according to the route, timing and frequency of the administration and the like. Depending on the symptom or disease state, a long-term treatment involving plural times of administration may be necessary.

The dose and administration schedule can be determined by a technique within the ordinary range known to those of ordinary skill in the art. In general, the prophylaxis or treatment is started from a dose lower than the optimal dose of the compound. Thereafter, the dose is gradually increased until the optimal effect is obtained under the circumstances. In the composition of the present invention, the daily dose of a VAP-1 inhibitor as an active ingredient is generally about 0.03 ng/kg body weight/day - about 300 mg/kg body weight/day, preferably about 0.003 μg/kg body weight/day - about 10 mg/kg body weight/day. Both a single administration and 2 to 4 times of administration per day can be employed before, between or after meals. In addition, the composition can be administered in a sustained manner.

The composition of the present invention preferably contains a "pharmaceutically acceptable carrier" and, as an active ingredient, a VAP-1 inhibitor in an amount sufficient to prophylactically or therapeutically treat an ophthalmic disease associated with hypoxia or ischemia or an amount sufficient to suppress angiogenesis. The carrier may be any as long as it is generally used as a medicament and is not particularly limited except when limited by physicochemical items for consideration (e.g., solubility, and lack of reactivity with the compound) and administration route.

The amount of the VAP-1 inhibitor in the composition of the present invention may vary depending on the formulation of the composition. It is generally 0.00001 - 10 wt%, preferably 0.001 - 5 wt%, more preferably 0.001 - 1 wt%, relative to the whole composition.

The administration form (dosage form) of the composition of the present invention is not particularly limited and can be administered in various forms to achieve a desired VAP-1 inhibitory action. The composition of the present invention can be formulated into an oral or parenteral preparation by using the composition of the present invention solely or in combination with a pharmaceutically acceptable additive such as carrier, diluent and the like. The characteristics and property of the preparation are determined by the solubility and chemical property of the active ingredient, selected administration route and standard pharmaceutical practice. Examples of the preparation to be used for oral administration include solid dosage forms (e.g., capsule, tablet, powder), liquid forms (e.g., solution or suspension) and the like. Examples of the preparation to be used for parenteral administration include injection, drip and eye drop, which are in the form of a sterile solution or suspension, eye ointment and the like. The solid oral preparation may contain conventional excipients (e.g., lactose, sucrose, magnesium stearate, resin, and like materials) and the like. The liquid oral preparation can contain various aromatic, colorant, preservative, stabilizer, solubilizer, suspending agent and the like. The parenteral preparation is, for example, an aseptic aqueous or nonaqueous solution or suspension, and can contain particular various preservatives, stabilizer, buffer agent, solubilizer, suspending agent and the like. Where necessary, the solution may be made isotonic by adding an additive such as saline or glucose.

The composition of the present invention may contain other pharmaceutically active compound as long as it does not inhibit the effect of the invention.

The composition of the present invention can be administered simultaneously with other pharmaceutically active compound as long as the effect of the present invention is not impaired. The "simultaneous administration" means administration of other pharmaceutically active compound simultaneously (e.g., in the same preparation or in different preparation) by the same or different administration route before or after administration of the composition of the present invention. Examples of other pharmaceutically active compound include corticosteroid, prednisone, methylprednisone, dexamethasone, triamcinolone acetonide or non-corticosteroid anti-inflammatory compound (e.g., ibuprofen or flurbiprofen). Similarly, vitamin and mineral (e.g., zinc, antioxidant such as carotenoid (e.g., xanthophyll carotenoid-like zeaxanthin or lutein)), trace nutrition and the like can be recited.

The present invention provides use of a VAP-1 inhibitor for the production of a medicament for the treatment of ophthalmic diseases associated with hypoxia or ischemia or a medicament for the suppression of angiogenesis.

In addition, the present invention provides a method of treating ophthalmic diseases associated with hypoxia or ischemia or a method of suppressing angiogenesis, which comprises a step of administering, to a subject in need of.the treatment, a VAP-1 inhibitor in an amount sufficient to treat the subject for the disease or suppress pathologic angiogenesis.

Furthermore, the present invention provides a commercial package comprising a composition for an ophthalmic disease associated with hypoxia or ischemia comprising a VAP-1 inhibitor as an active ingredient, and a written matter stating that the composition can or should be used for treating an ophthalmic disease associated with hypoxia or ischemia.

In addition, the present invention provides a commercial package comprising an angiogenesis inhibitor composition comprising a VAP-1 inhibitor as an active ingredient, and a written matter stating that the composition can or should be used for treating a disease requiring suppression of angiogenesis.

The present invention is explained in more detail in the following by referring to Examples (Production Example and Experimental Examples), which are not to be construed as limitative.

Examples

The starting compounds to be used in the following Production Example can be produced by a known method (W02004/067521, WO2006/011631, W02006/028269) and the like.

The Production Example of the VAP-1 inhibitor to be used in the present invention is shown below.

Production Example

Synthesis of N-{4-[2-(4-hydrazinocarbonylmethylphenyl)ethyl]-1,3-thiazol-2-yl}acetamide

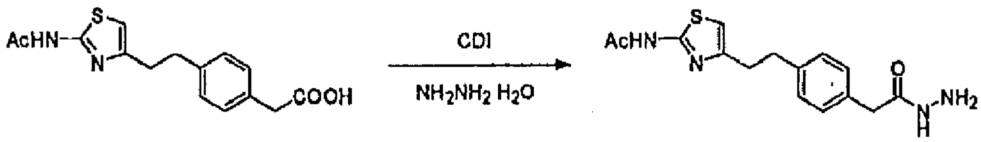


To a solution of 2-(4-{2-[2-(acetylamino)-1,3-thiazol-4-yl]ethyl}phenyl)acetic acid (913.1 mg, 3.00 mmol) in anhydrous dimethylformamide (7.5 ml) was added 1,1'-carbonyldiimidazole (729.7 mg, 4.50 mmol), and the mixture was stirred at 50°C for 1 hr. After cooling to room temperature, hydrazine monohydrate (0.73 ml, 15 mmol) was added, and the mixture was stirred at room temperature for 2 hr. Water (25 ml) was added, the mixture was stirred, and the produced solid was collected by filtration. The solid was washed three times with water, three times with ethyl acetate, and twice with tetrahydrofuran. The solid was dried under reduced pressure to give the title compound as a white solid (538.1 mg, 1.69 mmol, yield 56.3%).

melting point 200 - 202°C
$^1$H-NMR(200MHz,DMSO-d6): δ(ppm): 12.08(1H,brs), 9.18(1H,brs), 7.20-7.04(4H,m), 6.74(1H,s), 4.21(2H,brs), 3.40-3.25(2H,m), 3.00-2.80(4H,m), 2.11(3H,s) $^{13}$C-NMR(50MHz,DMSO-d6): δ(ppm): 169.8, 168.4, 157.6, 150.5, 139.6, 133.9, 129.0, 128.2, 107.5, 40.3, 34.3, 33.0, 22.7

Experimental Example 1

test substance 1: N-{4-[2-(9-hydrazinocarbonylmethylphenyl)ethyl]-1,3-thiazol-2-yl}acetamide (compound described in Production Example) medium: 1% polysorbate 80, 2.4% glycerol
test solution 1: Test substance 1 (0.01 mg) is dissolved in medium (1 mL).
test animal: C57BL/6N mouse (litter of 10)

A model experiment of retinopathy of prematurity, which is an ophthalmic disease associated with hypoxia or ischemia, was performed based on a quantitative analysis method of oxygen-induced retinopathy model mouse (Experimental Eye Research 84(2007) 529-536).

Preparation method of animal model

The date of birth of mouse was taken as day 0 of age. On day 7 of age, the mouse was placed in a high oxygen concentration breeding device together with the mother mouse and bred under high oxygen conditions (oxygen concentration 75±5%) up to day 12 of age. On day 12 of age, the high oxygen concentration breeding was terminated and

the mouse was immediately placed under general breeding with intraperitoneal administration of the medium or test solution 1 twice a day for 7 days. On day 19 of age, retina flat mount was prepared from 4 mice out of the litter.

Preparation method of retina flat mount

The mice were anesthetized by intraperitoneal administration of nembutal (1/10-fold diluted, 5 mg/mL) at 0.1 mL/mouse. The thoracic cavity was opened, and 1 mL of fluorescein isothiocyanate-dextran (FITC-dextran 20 mg/mL, FD2000S-1G, manufactured by SIGMA) was perfused from the heart. After perfusion, the eyeball was immediately isolated, and fixed with 4% para-formaldehyde for 3 - 4 hr. The eyeball was anatomized to isolate retina, which as developed on a slide glass to prepare a flat mount. The whole retina area, avascular region and abnormal angiogenesis region were encircled with a stylus pen (CTE-640/S1, WACOM FVO) under a fluorescence microscope (ECLIPSE 80i, manufactured by Nikon), and measured using an analysis soft (WinROOF ver.5.7.0, MITAMI Cor.), based on which the normal blood vessel region and abnormal angiogenesis region were calculated and compared.

normal blood vessel region (%): [{(whole retina area)-(area of avascular region)}/(whole retina area)]×100

abnormal angiogenesis region (%): {(abnormal angiogenesis region)/(whole retina area)}×100

Table 1 shows the ratio of normal blood vessel region and abnormal angiogenesis region in mouse retina.

Table 1

| | normal blood vessel region (%) | | abnormal angiogenesis region (%) | |
|---|---|---|---|---|
| | medium | test substance 1 | medium | test substance 1 |
| average | 89.96 | 93.44* | 12.17 | 7.04* |
| standard deviation | 0.85 | 1.21 | 1.27 | 1.62 |
| *; comparison with p<0.05 medium group (Student's t-test) sample numbers: medium 15 eyes; test substance 1 12 eyes | | | | |

Experimental Example 2

test substance 2: N-(4-{2-[4-(2-{[amino(imino)methyl]amino}ethyl)phenyl]ethyl}-1,3-thiazol-2-yl)acetamide (WO2006/011631, compound described in Production Example 25)

medium: 1% polysorbate 80, 2.4% glycerol

test solution 2: Test substance 2 (0.01 mg) is dissolved in medium (1 mL).

test animal: C57BL/6N mouse (litter of 4)

In the same manner as in Experimental Example 1 except that test solution 2 was administered once a day; test solution 2 was intraperitoneally administered to model mouse for 7 days, and the normal blood vessel region and abnormal angiogenesis region thereof were measured.

Table 2 shows the ratio of normal blood vessel region and abnormal angiogenesis region in mouse retina.

Table 2

| | normal blood vessel region (%) | | abnormal angiogenesis region (%) | |
|---|---|---|---|---|
| | medium | test substance 2 | medium | test substance 2 |
| average | 83.56 | 94.69* | 14.15 | 6.79* |

(continued)

| | normal blood vessel region (%) | | abnormal angiogenesis region (%) | |
|---|---|---|---|---|
| | medium | test substance 2 | medium | test substance 2 |
| standard deviation | 1.56 | 0.82 | 1.73 | 0.66 |
| *; comparison with $p<0.05$ medium group (Student's t-test) sample numbers: medium 14 eyes; test substance 2 18 eyes | | | | |

From the results of Table 1 and Table 2, a decrease in the avascular region and a suppressive effect on abnormal angiogenesis were observed in the test substances 1 and 2. Therefrom it was clarified that the test substances 1 and 2 are effective for the treatment of diseases associated with hypoxia or ischemia. In addition, the results show that the test substance group has an angiogenesis suppressive effect, since the abnormal angiogenesis region significantly decreased as compared to the medium group.

Industrial Applicability

According to the present invention, a novel composition for ophthalmic diseases associated with hypoxia or ischemia and an angiogenesis inhibitor composition can be provided.

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.

This application is based on a patent application No. 2007-166989 filed in Japan (filing date: June 25, 2007), the contents of which are incorporated in full herein by this reference.

THE FOLLOWING ARE ADDITIONAL ASPECTS OF THE PRESENT INVENTION:

1. A composition for an ophthalmic disease associated with hypoxia or ischemia, comprising a VAP-1 inhibitor as an active ingredient.

2. The composition according to 1, wherein said VAP-1 inhibitor is a compound represented by the following formula (I):

$$R^1\text{-NH-X-Y-Z} \qquad (I)$$

wherein
$R^1$ is acyl;
X is a divalent residue induced from optionally substituted thiazole;
Y is the formula: $Y^1$-$Y^2$-$Y^3$
wherein $Y^1$ is a bond, lower alkylene, lower alkenylene, lower alkynylene, -($CH_2$)n-O-, -$(CH_2)_n$-NH-, -$(CH_2)_n$-CO- or - $(CH_2)_n$-SO2- (wherein n is an integer of 0 to 6);
$Y^2$ is a bond, -O-, -NH-, -CO- or -$SO_2$-;
$Y^3$ is a bond, lower alkylene, lower alkenylene or lower alkynylene, provided that when $Y^1$ is -$(CH_2)_n$-O-, then $Y^2$ is not -O-, -NH- or -$SO_2$-, when $Y^1$ is -$(CH_2)_n$-NH-, then $Y^2$ is not -O- or -NH-, when $Y^1$ is -$(CH_2)_n$-CO-, then $Y^2$ is not -CO-, and when $Y^1$ is -$(CH_2)_n$-$SO_2$-, then $Y^2$ is not -O- or -$SO_2$-(wherein n is as defined above);
Z is the formula:

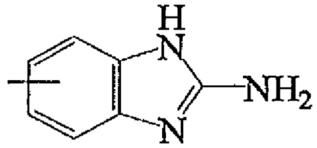 , 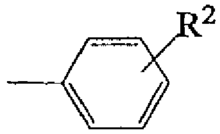 or 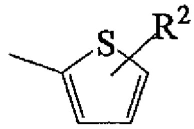

wherein $R^2$ is the formula: -A-B-D-E
wherein
A is a bond, lower alkylene, -$NR^{2a}$- or -$SO_2$- wherein $R^{2a}$ is hydrogen, lower alkyl or acyl;

B is a bond, lower alkylene, -CO- or -O-;
D is a bond, lower alkylene, $-NR^{2b}-$ or $-CH_2NH-$ wherein $R^{2b}$ is hydrogen, lower alkyl, alkoxycarbonyl or acyl; and
E is optionally substituted amino, $-N=CH_2$,

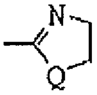 or 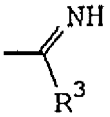

wherein Q is -S- or -NH-; $R^3$ is hydrogen, lower alkyl, lower alkylthio or $-NH-R^4$ wherein $R^4$ is hydrogen, $-NH_2$ or lower alkyl, or a derivative thereof, or a pharmaceutically acceptable salt thereof.

3. The composition according to 2, wherein, in the formula (I), Z is the formula (II):

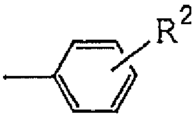 (I I)

wherein
$R^2$ is the formula:

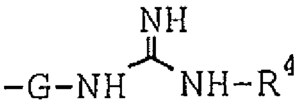

wherein G is a bond, $-NHCOCH_2-$ or lower alkylene; $R^4$ is hydrogen, $-NH_2$ or lower alkyl;
$-NH_2$; $-CH_2NH_2$; $-CH_2ONH_2$; $-CH_2ON=CH_2$;

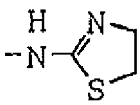 ; 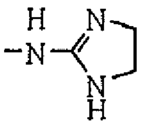 ; 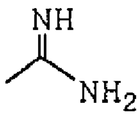 ; 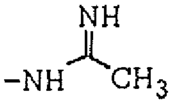 or 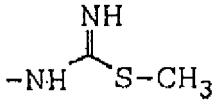 .

4. The composition according to 3, wherein, in the formula (II), $R^2$ is the formula:

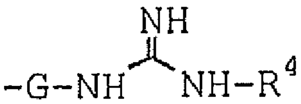

wherein G is a bond, $-NHCOCH_2-$ or lower alkylene, and $R^4$ is hydrogen, $-NH_2$ or lower alkyl;
$-NH_2$; $-CH_2NH_2$; $-CH_2ONH_2$; $-CH_2ON=CH_2$;

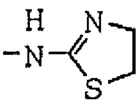 ; 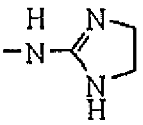 ; 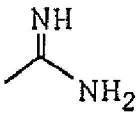 ; 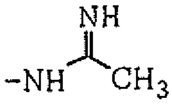 or 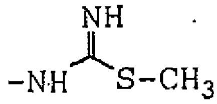 .

5. The composition according to 4, wherein, in the formula (I), $R^2$ is the following formula (III):

J-L-M (III)

wherein

J is $-NR^{2a}-$, $-NR^{2a}-CO-$, $-(CH_2)_n-$ or $-(CH_2)_nCO-$ wherein $R^{2a}$ is hydrogen, lower alkyl, or acyl; n is an integer of 0 to 6;
L is $-NR^{2b}-$ wherein $R^{2b}$ is hydrogen, lower alkyl, alkoxycarbonyl or acyl; and
M is optionally substituted amino.

6. The composition according to 5, wherein, in the formula (III), J-L-M is $-CO-NH-NH_2$, $-CH_2-CO-NH-NH_2$, $-CH_2-CO-NH-NH-CH_3$, $-CH_2-CO-N(CH_3)-NH_2$, $-CH_2-CO-NH-NH-C_2H_5$, $-CH_2-CO-NH-N(CH_3)_2$, $-(CH_2)_2-CO-NH-NH_2$, $-NH-CO-NH-NH_2$, $-NH-NH_2$, $-CH_2-NH-NH_2$, $-(CH_2)_2-NH-NH_2$ or $-(CH_2)_3-NH-NH_2$.

7. The composition according to any one of 2 to 6, wherein, in the formula (I), $R^1$ is alkylcarbonyl, and X is a divalent residue induced from thiazole optionally substituted by methylsulfonylbenzyl.

8. The composition according to 1, wherein said VAP-1 inhibitor is N-{4-[2-(4-hydrazinocarbonylmethylphenyl)ethyl]-1,3-thiazol-2-yl}acetamide;
or a derivative thereof;
or a pharmaceutically acceptable salt thereof.

9. The composition according to 1, wherein said VAP-1 inhibitor is N-(4-{2-[4-(2-{[amino(imino)methyl]amino}ethyl) phenyl]ethyl}-1,3-thiazol-2-yl)acetamide;
or a derivative thereof;
or a pharmaceutically acceptable salt thereof.

10. The composition according to any one of 1 to 9, wherein said ophthalmic disease is ischemic retinopathy or ischemic optic neuropathy.

11. The composition according to any one of 1 to 9, wherein said ophthalmic disease is retinopathy of prematurity, proliferative diabetic retinopathy, polypoid choroidal vasculopathy, retinal angiomatous proliferation, retinal artery occlusion, retinal vein occlusion, Coats' disease, familial exudative vitreoretinopathy, pulseless disease (Takayasu disease), Eales' disease, antiphospholipid syndrome, leukemiaretinopathy, blood hyperviscosity syndrome, macroglobulinemia, interferon retinopathy, hypertensive retinopathy, radiation retinopathy or cornea epithelial stem cell deficiency.

12. The composition according to 11, wherein said ophthalmic disease is retinopathy of prematurity.

13. Use of a VAP-1 inhibitor for the production of a medicament for the treatment of an ophthalmic disease associated with hypoxia or ischemia.

14. A method of treating an ophthalmic disease associated with hypoxia or ischemia, comprising a step of administering, to a subject in need of the treatment, a VAP-1 inhibitor in an amount sufficient to treat the subject for the disease.

15. A commercial package comprising the composition according to any one of 1 to 12, and a written matter stating that the composition can or should be used for treating an ophthalmic disease associated with hypoxia or ischemia.

16. An angiogenesis inhibitor composition comprising a VAP-1 inhibitor as an active ingredient.

17. The composition according to 16, wherein said VAP-1 inhibitor is a compound represented by the following formula **(I):**

$$R^1\text{-NH-X-Y-Z} \qquad \text{(I)}$$

wherein
$R^1$ is acyl;
X is a divalent residue induced from optionally substituted thiazole;
Y is the formula: $Y^1$-$Y^2$-$Y^3$
wherein $Y^1$ is a bond, lower alkylene, lower alkenylene, lower alkynylene, $-(CH_2)_n-O-$, $-(CH_2)_n-NH-$, $-(CH_2)_n-CO-$ or $-(CH_2)_n-SO_2-$ (wherein n is an integer of 0 to 6);
$Y^2$ is a bond, -O-, -NH-, -CO- or $-SO_2-$;
$Y^3$ is a bond, lower alkylene, lower alkenylene or lower alkynylene, provided that when $Y^1$ is $-(CH_2)_n-O-$, then $Y^2$ is

not -O-, -NH- or $-SO_2-$, when $Y^1$ is $-(CH_2)_n-NH-$, then $Y^2$ is not -O- or -NH-, when $Y^1$ is $-(CH_2)_n-CO-$, then $Y^2$ is not -CO-, and when $Y^1$ is $-(CH_2)_n$-SO2-, then $Y^2$ is not -O- and $-SO_2-$(wherein n is as defined above);
Z is the formula:

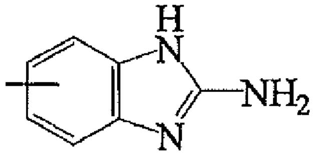
,
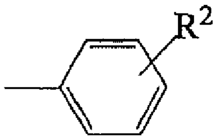
or
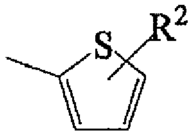

wherein $R^2$ is the formula: -A-B-D-E
wherein
A is a bond, lower alkylene, $-NR^{2a}-$ or $-SO_2-$ wherein $R^{2a}$ is hydrogen, lower alkyl or acyl;
B is a bond, lower alkylene, -CO- or -O-;
D is a bond, lower alkylene, $-NR^{2b}-$ or $-CH_2NH-$ wherein $R^{2b}$ is hydrogen, lower alkyl, alkoxycarbonyl or acyl; and
E is optionally substituted amino, $-N=CH_2$,

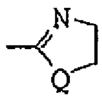
or
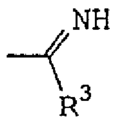

wherein
Q is -S- or -NH-;
$R^3$ is hydrogen, lower alkyl, lower alkylthio or $-NH-R^4$
wherein $R^4$ is hydrogen, $-NH_2$ or lower alkyl, or a derivative thereof, or a pharmaceutically acceptable salt thereof.

18. The composition according to 17, wherein, in the formula (I), Z is the formula (II):

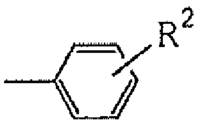
(I I)

wherein
$R^2$ is the formula:

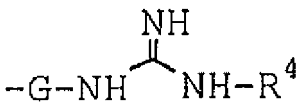

wherein G is a bond, $-NHCOCH_2-$ or lower alkylene; $R^4$ is hydrogen, $-NH_2$ or lower alkyl;
$-NH_2$; $-CH_2NH_2$; $-CH_2ONH_2$; $-CH_2ON=CH_2$;

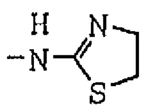
;
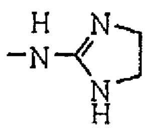
;
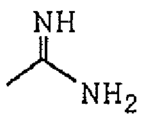
;
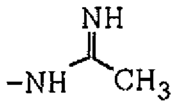
or
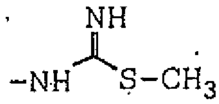
.

19. The composition according to 18, wherein, in the formula (II), $R^2$ is the formula:

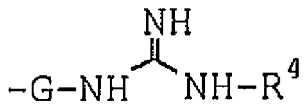

wherein G is a bond, $-NHCOCH_2-$ or lower alkylene, and $R^4$ is hydrogen, $-NH_2$ or lower alkyl;
$-NH_2$; $-CH_2NH_2$; $-CH_2ONH_2$; $-CH_2ON=CH_2$;

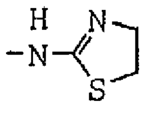 ; 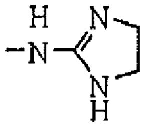 ; 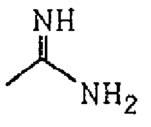 ; 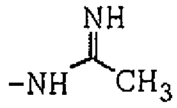 or 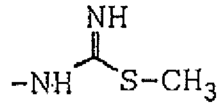 .

20. The composition according to 19, wherein, in the formula (I), $R^2$ is the following formula (III):

J-L-M (III)

wherein
J is $-NR^{2a}-$, $-NR^{2a}-CO-$, $-(CH_2)_n-$ or $-(CH_2)_nCO-$ wherein $R^{2a}$ is hydrogen, lower alkyl or acyl; and n is an integer of 0 to 6; L is $-NR^{2b}-$ wherein $R^{2b}$ is hydrogen, lower alkyl, alkoxycarbonyl or acyl; and
M is optionally substituted amino.

21. The composition according to 20, wherein, in the formula (III), J-L-M is $-CO-NH-NH_2$, $-CH_2-CO-NH-NH_2$, $-CH_2-CO-NH-NH-CH_3$, $-CH_2-CO-N(CH_3)-NH_2$, $-CH_2-CO-NH-NH-C_2H_5$, $-CH_2-CO-NH-N(CH_3)_2$, $-(CH_2)_2-CO-NH-NH_2$, $-NH-CO-NH-NH_2$, $-NH-NH_2$, $-CH_2-NH-NH_2$, $-(CH_2)_2-NH-NH_2$ or $-(CH_2)_3-NH-NH_2$.

22. The composition according to any one of 17 to 21, wherein, in the formula (I), $R^1$ is alkylcarbonyl, and X is a divalent residue induced from thiazole optionally substituted by methylsulfonylbenzyl.

23. The composition according to 16, wherein said VAP-1 inhibitor is N-{4-[2-(4-hydrazinocarbonylmethylphenyl)ethyl]-1,3-thiazol-2-yl}acetamide;
or a derivative thereof;
or a pharmaceutically acceptable salt thereof.

24. The composition according to 16, wherein said VAP-1 inhibitor is N-(4-{2-[4-(2-{[amino(imino)methyl]amino}ethyl)phenyl]ethyl}-1,3-thiazol-2-yl)acetamide;
or a derivative thereof;
or a pharmaceutically acceptable salt thereof.

25. The composition according to any one of 16 to 24, wherein the blood vessel is an ocular blood vessel.

26. Use of a VAP-1 inhibitor for the production of a medicament for the suppression of angiogenesis.

27. A method of suppressing angiogenesis, comprising a step of administering, to a subject in need of a treatment, a VAP-1 inhibitor in an amount sufficient to suppress angiogenesis in the subject.

28. A commercial package comprising the composition according to any one of 16 to 25, and a written matter stating that the composition can or should be used for treating a disease requiring suppression of angiogenesis.

## Claims

**1.** A composition comprising a VAP-1 inhibitor as an active ingredient for use in inhibition of angiogenesis or for use in treatment of ophthalmic disease associated with hypoxia or ischemia.

**2.** Composition for use according to claim 1, wherein said VAP-1 inhibitor is a compound represented by the following formula (I):

**$R^1$-NH-X-Y-Z (I)**

wherein
$R^1$ is acyl;
X is a divalent residue derived from optionally substituted thiazole;
Y is the formula: $Y^1$-$Y^2$-$Y^3$
wherein $Y^1$ is a bond, lower alkylene, lower alkenylene, lower alkynylene, $-(CH_2)_n$-O-, $-(CH_2)_n$-NH-, $-(CH_2)_n$-CO- or $-(CH_2)_n-SO_2$-(wherein n is an integer of 0 to 6);
$Y^2$ is a bond, -O-, -NH-, -CO- or $-SO_2$-;
$Y^3$ is a bond, lower alkylene, lower alkenylene or lower alkynylene, provided that when $Y^1$ is $-(CH_2)_n$-O-, then $Y^2$ is not -O-, -NH- or $-SO_2$-, when $Y^1$ is $-(CH_2)_n$-NH-, then $Y^2$ is not -O- or -NH-, when $Y^1$ is - $(CH_2)_n$-CO-, then $Y^2$ is not -CO-, and when $Y^1$ is $-(CH_2)_n-SO_2$-, then $Y^2$ is not -O- or $-SO_2$-(wherein n is as defined above);
Z is the formula:

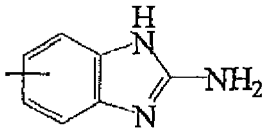 , 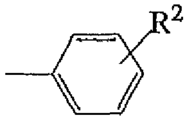 or 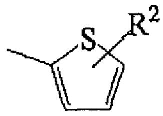

wherein $R^2$ is the formula: -A-B-D-E
wherein
A is a bond, lower alkylene, $-NR^{2a}$- or $-SO_2$- wherein $R^{2a}$ is hydrogen, lower alkyl or acyl;
B is a bond, lower alkylene, -CO- or -O-;
D is a bond, lower alkylene, $-NR^{2b}$- or $-CH_2NH$- wherein $R^{2b}$ is hydrogen, lower alkyl, alkoxycarbonyl or acyl; and
E is optionally substituted amino, $-N=CH_2$,

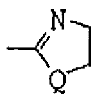 or 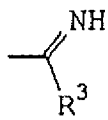

wherein
Q is -S- or -NH-;
$R^3$ is hydrogen, lower alkyl, lower alkylthio or $-NH-R^4$
wherein $R^4$ is hydrogen, $-NH_2$ or lower alkyl,
or a derivative thereof, or a pharmaceutically acceptable salt thereof.

3. Composition for use according to claim 2, wherein, in the formula (I), Z is the formula (II):

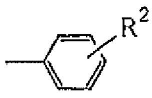 (I I)

wherein
$R^2$ is the formula:

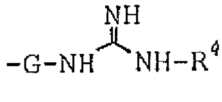

wherein G is a bond, $-NHCOCH_2$- or lower alkylene; $R^4$ is hydrogen, $-NH_2$ or lower alkyl;
$-NH_2$; $-CH_2NH_2$; $-CH_2ONH_2$; $-CH_2ON=CH_2$;

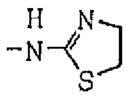 ; 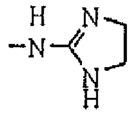 ; 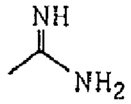 ; 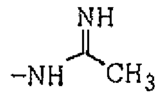 or 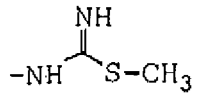 .

**4.** Composition for use according to claim 3, wherein, in the formula (II), $R^2$ is the formula:

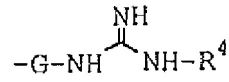

wherein G is a bond, $-NHCOCH_2-$ or lower alkylene; and $R^4$ is hydrogen, $-NH_2$ or lower alkyl; $-NH_2$; $-CH_2NH_2$; $-CH_2ONH_2$; $-CH_2ON=CH_2$;

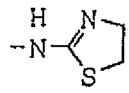 ; 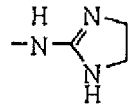 ; 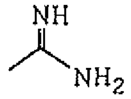 ; 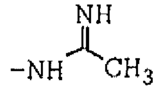 or 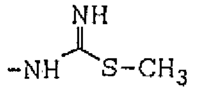 .

**5.** Composition for use according to claim 2, wherein, in the formula (I), $R^2$ is the following formula (III):

J-L-M (III)

wherein
J is $-NR^{2a}-$, $-NR^{2a}-CO-$, $-(CH_2)_n-$ or $-(CH_2)_nCO-$ wherein $R^{2a}$ is hydrogen, lower alkyl, or acyl; n is an integer of 0 to 6;
L is $-NR^{2b}-$ wherein $R^{2b}$ is hydrogen, lower alkyl, alkoxycarbonyl or acyl; and
M is optionally substituted amino.

**6.** Composition for use according to claim 5, wherein, in the formula (III), J-L-M is -CO-NH-$NH_2$, $-CH_2$-CO-NH-$NH_2$, $-CH_2$-CO-NH-NH-$CH_3$, $-CH_2$-CO-N($CH_3$)-$NH_2$, $-CH_2$-CO-NH-NH-$C_2H_5$, $-CH_2$-CO-NH-N$(CH_3)_2$, $-(CH_2)_2$-CO-NH-$NH_2$, -NH-CO-NH-$NH_2$, -NH-$NH_2$, $-CH_2$-NH-$NH_2$, $-(CH_2)_2$-NH-$NH_2$ or $-(CH_2)_3$-NH-$NH_2$.

**7.** Composition for use according to any one of claims 2 to 6, wherein, in the formula (I), $R^1$ is alkylcarbonyl, and X is a divalent residue derived from thiazole optionally substituted by methylsulfonylbenzyl.

**8.** Composition for use according to claim 1, wherein said VAP-1 inhibitor is
N-{4-[2-(4-hydrazinocarbonylmethylphenyl)ethyl]-1,3-thiazol-2-yl} acetamide;
or a derivative thereof;
or a pharmaceutically acceptable salt thereof.

**9.** Composition for use according to claim 1, wherein said VAP-1 inhibitor is
N-(4-{2-[4-(2-{[amino(imino)methyl]amino}ethyl)phenyl]ethyl}-1,3-thiazol-2-yl)acetamide;
or a derivative thereof;
or a pharmaceutically acceptable salt thereof.

**10.** Composition for use according to any one of claims 1 to 9, wherein:

- said ophthalmic disease is ischemic retinopathy or ischemic optic neuropathy; or
- said ophthalmic disease is retinopathy of prematurity, proliferative diabetic retinopathy, polypoid choroidal vasculopathy, retinal angiomatous proliferation, retinal artery occlusion, retinal vein occlusion, Coats' disease, familial exudative vitreoretinopathy, pulseless disease (Takayasu disease), Eales' disease, antiphospholipid syndrome, leukemiaretinopathy, blood hyperviscosity syndrome, macroglobulinemia, interferon retinopathy, hypertensive retinopathy, radiation retinopathy or cornea epithelial stem cell deficiency, wherein said ophthalmic disease is preferably retinopathy of prematurity.

**11.** Composition for use according to any one of claims 1 to 9, wherein the blood vessel in said angiogenesis is an

ocular blood vessel.

**12.** Use of a VAP-1 inhibitor for the production of a medicament (i) for the suppression of angiogenesis or (ii) for the treatment of an ophthalmic disease associated with hypoxia or ischemia.

**13.** A compound represented by the following formula (I):

$R^1$-NH-X-Y-Z (I)

wherein
$R^1$ is acyl;
X is a divalent residue derived from optionally substituted thiazole;
Y is the formula: $Y^1$-$Y^2$-$Y^3$
wherein $Y^1$ is a bond, lower alkylene, lower alkenylene, lower alkynylene, $-(CH_2)_n$-O-, $-(CH_2)_n$-NH-, $-(CH_2)_n$-CO- or $-(CH_2)_n-SO_2$-(wherein n is an integer of 0 to 6);
$Y^2$ is a bond, -O-, -NH-, -CO- or $-SO_2-$;
$Y^3$ is a bond, lower alkylene, lower alkenylene or lower alkynylene, provided that when $Y^1$ is $-(CH_2)_n$-O-, then $Y^2$ is not -O-, -NH- or $-SO_2-$, when $Y^1$ is $-(CH_2)_n$-NH-, then $Y^2$ is not -O- or -NH-, when $Y^1$ is $-(CH_2)_n$-CO-, then $Y^2$ is not -CO-, and when $Y^1$ is $-(CH_2)_n-SO_2-$, then $Y^2$ is not -O- or $-SO_2$-(wherein n is as defined above);
Z is the formula:

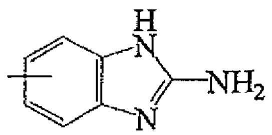 , 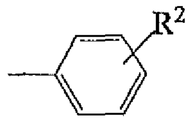 or 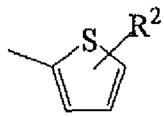

wherein $R^2$ is the following formula (III):

J-L-M (III)

wherein
J is $-NR^{2a}-$, $-NR^{2a}$-CO-, $-(CH_2)_n$- or - $(CH_2)_n$CO- wherein $R^{2a}$ is hydrogen, lower alkyl, or acyl; n is an integer of 0 to 6;
L is $-NR^{2b}-$ wherein $R^{2b}$ is hydrogen, lower alkyl, alkoxycarbonyl or acyl; and
M is optionally substituted amino,
or a derivative thereof;
or a pharmaceutically acceptable salt thereof.

**14.** The compound according to claim 13, wherein:

(i) in the formula (III), J-L-M is -CO-NH-$NH_2$, $-CH_2$-CO-NH-$NH_2$, $-CH_2$-CO-NH-NH-$CH_3$, $-CH_2$-CO-N($CH_3$)-$NH_2$, $-CH_2$-CO-NH-NH-$C_2H_5$, $-CH_2$-CO-NH-N$(CH_3)_2$, $-(CH_2)_2$-CO-NH-$NH_2$, -NH-CO-NH-$NH_2$, -NH-$NH_2$, $-CH_2$-NH-$NH_2$, $-(CH_2)_2$-NH-$NH_2$ or $-(CH_2)_3$-NH-$NH_2$; or
(ii) in the formula (I), $R^1$ is alkylcarbonyl, and X is a divalent residue derived from thiazole optionally substituted by methylsulfonylbenzyl, and Z is the formula

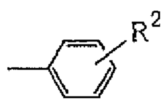

wherein $R^2$ is as defined in claim 13;

**15.** The compound according to claim 13, wherein the compound is N-{4-[2-(4-hydrazinocarbonylmethylphenyl)ethyl]-1,3-thiazol-2-yl} acetamide or a derivative thereof or a pharmaceutically acceptable salt thereof.

Europäisches Patentamt
European Patent Office
Office européen des brevets

# EUROPEAN SEARCH REPORT

Application Number
EP 13 15 7478

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br><br><br>Y | WO 2006/028269 A2 (ASTELLAS PHARMA INC [JP]; INOUE TAKAYUKI [JP]; TOJO TAKASHI [JP]; MORI) 16 March 2006 (2006-03-16)<br>* claims 1-12 *<br>----- | 1-9, 11-15<br><br><br>1-15 | INV.<br>A61K45/00<br>A61K31/426<br>A61P27/02 |
| Y | GOKTURK C ET AL: "Studies on semicarbazide-sensitive amine oxidase in patients with diabetes mellitus and in transgenic mice",<br>BIOCHIMICA ET BIOPHYSICA ACTA (BBA) - PROTEINS & PROTEOMICS, ELSEVIER LNKD-DOI:10.1016/S1570-9639(03)00064-5,<br>vol. 1647, no. 1-2,<br>11 April 2003 (2003-04-11), pages 88-91, XP004417968,<br>ISSN: 1570-9639<br>* paragraph entitled "5. Conclusions and perspectives" *<br>----- | 1-15 | |
| A | GARPENSTRAND H ET AL: "Serum semicarbazide-sensitive amine oxidase (SSAO) activity correlates with VEGF in non-small-cell lung cancer patients",<br>MEDICAL ONCOLOGY, SCIENCE AND TECHNOLOGY LETTERS, NORTHWOOD, GB LNKD-DOI:10.1385/MO:21:3:241,<br>vol. 21, no. 3,<br>1 September 2004 (2004-09-01), pages 241-250, XP009121090,<br>ISSN: 1357-0560<br>* paragraph entitled "Discussion"; figure 4 *<br>-----<br>-/-- | 1-15 | TECHNICAL FIELDS SEARCHED (IPC)<br>A61K |

The present search report has been drawn up for all claims

| Place of search | Date of completion of the search | Examiner |
|---|---|---|
| Munich | 3 April 2013 | Borst, Markus |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 15 7478

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHIKARAISHI ET AL: "New quantitative analysis, using high-resolution images, of oxygen-induced retinal neovascularization in mice", EXPERIMENTAL EYE RESEARCH, ACADEMIC PRESS LTD, LONDON, vol. 84, no. 3, 7 February 2007 (2007-02-07), pages 529-536, XP005938164, ISSN: 0014-4835, DOI: 10.1016/J.EXER.2006.11.007 * page 529-530, paragraph entitled "1. Introduction" * | 1-15 | |
| X | WO 2004/087138 A1 (SUCAMPO AG [CH]; FUJISAWA PHARMACEUTICAL CO [JP]; UENO RYUJI [US]; NAG) 14 October 2004 (2004-10-14) | 1-15 | |
| Y | * claim 1, 4-10, 21, 24-30 * | 1-15 | |
| Y | ESZTER DURA: "THE POTENTIAL ROLE OF THE SEMICARBAZIDE-SENSITIVE AMINE OXIDASE ACTIVITY IN THE DEVELOPMENT OF DIABETIC RETINOPATHY", LEGE ARTIS MEDICINAE, LIT. MED, BUDAPEST, HU, vol. 16, no. 7, 1 January 2006 (2006-01-01), pages 637-642, XP009121052, ISSN: 0866-4811 * abstract * | 1-15 | TECHNICAL FIELDS SEARCHED (IPC) |
| A | WO 2004/067521 A1 (FUJISAWA PHARMACEUTICAL CO [JP]; INOUE TAKAYUKI [JP]; TOJO TAKASHI [JP] 12 August 2004 (2004-08-12) * claim 1-23 * | 1-15 | |
| | -/-- | | |

The present search report has been drawn up for all claims

1

| Place of search | Date of completion of the search | Examiner |
|---|---|---|
| Munich | 3 April 2013 | Borst, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 15 7478

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2006/011631 A2 (ASTELLAS PHARMA INC [JP]; INOUE TAKAYUKI [JP]; TOJO TAKASHI [JP]; MORI) 2 February 2006 (2006-02-02)<br>* claims 1-21 *<br>----- | 1-15 | |
| X,P | WO 2008/066145 A1 (R TECH UENO LTD [JP]; MATSUKAWA TATSUYA [JP]; MASUZAKI KAZUHIRO [JP];) 5 June 2008 (2008-06-05)<br>* claims 1-14 *<br>----- | 1-15 | |

TECHNICAL FIELDS SEARCHED (IPC)

The present search report has been drawn up for all claims

1

| Place of search | Date of completion of the search | Examiner |
|---|---|---|
| Munich | 3 April 2013 | Borst, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 15 7478

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2013

| Patent document cited in search report | Publication date | Patent family member(s) | Publication date |
|---|---|---|---|
| WO 2006028269 A2 | 16-03-2006 | CA 2579889 A1 | 16-03-2006 |
| | | EP 1791835 A2 | 06-06-2007 |
| | | JP 2008512346 A | 24-04-2008 |
| | | US 2008015202 A1 | 17-01-2008 |
| | | WO 2006028269 A2 | 16-03-2006 |
| WO 2004087138 A1 | 14-10-2004 | CA 2520957 A1 | 14-10-2004 |
| | | CN 1794988 A | 28-06-2006 |
| | | EP 1608365 A1 | 28-12-2005 |
| | | JP 4758337 B2 | 24-08-2011 |
| | | JP 2006522110 A | 28-09-2006 |
| | | KR 20060023522 A | 14-03-2006 |
| | | KR 20120045062 A | 08-05-2012 |
| | | US 2006229346 A1 | 12-10-2006 |
| | | US 2008119462 A1 | 22-05-2008 |
| | | WO 2004087138 A1 | 14-10-2004 |
| WO 2004067521 A1 | 12-08-2004 | AR 042941 A1 | 06-07-2005 |
| | | CA 2514573 A1 | 12-08-2004 |
| | | EP 1587800 A1 | 26-10-2005 |
| | | JP 4650412 B2 | 16-03-2011 |
| | | JP 2006516611 A | 06-07-2006 |
| | | KR 20050095875 A | 04-10-2005 |
| | | KR 20120030601 A | 28-03-2012 |
| | | TW I336696 B | 01-02-2011 |
| | | US 2004259923 A1 | 23-12-2004 |
| | | US 2006128770 A1 | 15-06-2006 |
| | | US 2006276521 A1 | 07-12-2006 |
| | | WO 2004067521 A1 | 12-08-2004 |
| WO 2006011631 A2 | 02-02-2006 | AT 427941 T | 15-04-2009 |
| | | CA 2575411 A1 | 02-02-2006 |
| | | CN 101031555 A | 05-09-2007 |
| | | EP 1786792 A2 | 23-05-2007 |
| | | ES 2324922 T3 | 19-08-2009 |
| | | JP 4978464 B2 | 18-07-2012 |
| | | JP 2008508188 A | 21-03-2008 |
| | | KR 20070050932 A | 16-05-2007 |
| | | US 2007254931 A1 | 01-11-2007 |
| | | WO 2006011631 A2 | 02-02-2006 |
| WO 2008066145 A1 | 05-06-2008 | TW 200835687 A | 01-09-2008 |
| | | WO 2008066145 A1 | 05-06-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004067521 A
- WO 2004087138 A
- WO 2006011631 A
- WO 2006028269 A
- JP 2007166989 A


### Non-patent literature cited in the description


- Protective Groups in Organic Synthesis. John Wiley and Sons, 1999
- *Experimental Eye Research,* 2007, vol. 84, 529-536